(19) Europäisches Patentamt
European Patent Office
Office européen des brevets

(11) Numéro de publication : **0 363 230 B1**

# (12) FASCICULE DE BREVET EUROPEEN

(45) Date de publication du fascicule du brevet : **08.07.92 Bulletin 92/28**

(51) Int. Cl.[5] : **A61K 31/365,** C07D 307/58

(21) Numéro de dépôt : **89402004.9**

(22) Date de dépôt : **12.07.89**

## (54) Utilisation d'acétogénines en thérapeutique en tant que substances antiparasitaires.

(30) Priorité : **18.07.88 FR 8809674**

(43) Date de publication de la demande : **11.04.90 Bulletin 90/15**

(45) Mention de la délivrance du brevet : **08.07.92 Bulletin 92/28**

(84) Etats contractants désignés : **AT BE CH DE ES FR GB GR IT LI LU NL SE**

(56) Documents cités :
**FR-A- 2 617 843**
**US-A- 4 721 727**
**Journal of Organic Chemistry, vol. 47, no. 26, 1982, pages 3151-3153, American Chemical Society, Easton, US.**
**Journal of Natural Products, vol. 47, no. 4, 1984, pages 652-657, The American Society of Pharmacognosy, Columbus, Ohio, US.**
**Tetrahedron Letters, vol. 25, no. 30, 1985, pages 3199-3202, Pergamon Press Ltd., Oxford, GB.**

(56) Documents cités :
**Canadian Journal of Chemistry, vol. 65, no. 6, juin 1987, pages 1433- 1435, Ottawa, CA.**
**Journal of Organic Chemistry, vol. 53, no. 23, 1988, pages 5578-5580, American Chemical Society, Easton, US.**

(73) Titulaire : **LABORATOIRES DEBAT Société anonyme dite:**
**60 rue de Monceau**
**F-75008 Paris (FR)**

(72) Inventeur : **Cave, André**
**29, rue Ravon**
**F-92340 Bourg La Reine (FR)**
Inventeur : **Hocquemiller, Reynald**
**9, rue d'Arpajon**
**F-91470 Limours (FR)**
Inventeur : **Laprevote, Olivier**
**2, Clos Saint Victor**
**F-78220 Viroflay (FR)**

(74) Mandataire : **Clisci, Serge et al**
**S.A. FEDIT-LORIOT CONSEILS EN PROPRIETE INDUSTRIELLE 38, avenue Hoche**
**F-75008 Paris (FR)**

EP 0 363 230 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

### DOMAINE DE L'INVENTION

La présente invention concerne une nouvelle utilisation en thérapeutique des composés qui appartiennent à la famille des acétogénines en tant que substances antiparasitaires.

Les acétogénines décrites ci-après sont des produits principalement extraits de plantes appartenant à la famille des annonacées et notamment des genres Rollinia, Uvaria, Annona et Asiminia.

### ART ANTERIEUR

On sait que l'on a déjà décrit dans la littérature les propriétés cytotoxiques et antitumorales de lactones d'acide gras comportant dans leur molécule un groupe bis(tétrahydrofurannyle) et répondant à la formule

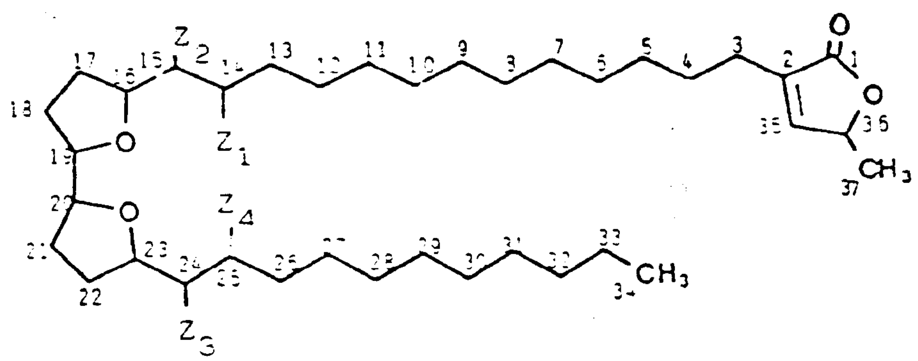

(II)

On connait en particulier une acétogénine de formule II [où $Z_1$=$Z_4$=H, $Z_2$=OH et $Z_3$=$OCOCH_3$] décrite par JOLAD et al., J. Org. Chem., 47, pages 3151-3153, (1982) qui a été extraite de Uvaria accuminata et a été dénommée "uvaricine".

On connait également la "désacétyluvaricine", qui est une acétogénine de formule II [où $Z_1$=$Z_4$=H et $Z_2$=$Z_3$=OH] décrite par JOLAD et al., J. Nat. Prod. 48 (N° 4), pages 644-645, (1985).

De DABRAH et al., Phytochemistry, 23 (N° 9), pages 2013-2016, (1984), on connait deux stéréoisomères de formule II (appelés "rollinine" et "isorollinine") [où $Z_1$=H et $Z_2$=$Z_3$=$Z_4$=OH], extraits de Rollinia papilionella et présentés comme ayant une activité cytotoxique.

On connait encore de DABRAH et al., J. Nat. Prod., 47 (N°4), pages 652-657, (1984), la "rollinone" qui est un composé de formule II [où $Z_4$=H, $Z_2$=$Z_3$=OH, $Z_1$ représente une fonction oxo, et, dans le cycle lactone, la liaison $C_1$-$C_{35}$ est totalement saturée].

On connait aussi de CORTES et al., Tetrahedron Letters 25 (N°30), pages 3199-3202 (1984) des acétogénines extraites de Annona cherimolia de formule

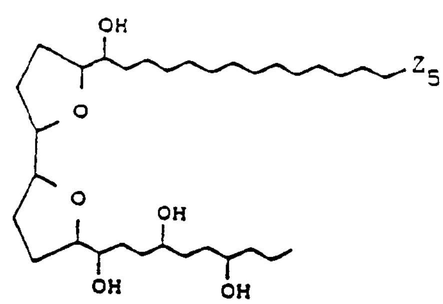

(III)

où $Z_5$ est un reste 3-(2-oxo-5-méthyl-2,5-dihydrofurannyle) [produit dénommé "chérimoline"] ou 3-(2-oxo-5-méthyl-2,3,4,5-tétrahydrofurannyle) [produit dénommé "dihydrochérimoline"] et qui sont signalées comme ayant des propriétés antimicrobiennes vis-à-vis des bactéries $G^-$ et des Candida.

On sait enfin que le brevet américain US-A-4 721 727 propose l'utilisation en agriculture en tant qu'agents pesticides, d'une part, et en thérapeutique notamment vétérinaire en tant qu'agents parasiticides [voir colonne 4 lignes 49-51 (où il est fait mention de véhicules inertes pharmaceutiquement acceptables), colonne 5 lignes

23-25 (où est signalé le traitement de parasites internes de l'animal) et exemples 3-4 colonnes 9-10 (où il est fait état d'essais sur des nématodes en suspension dans un milieu aqueux)], d'autre part, des acétogénines de formule

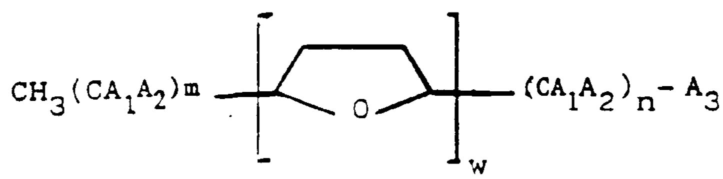 (IV)

dans laquelle
le couple ($A_1A_2$) est choisi parmi les couples (H,H), (H,OH), (H,OAc) et (=O), avec la condition que le nombre total de substituants oxy des groupes $A_1$ et $A_2$ ne soit pas supérieur à 6 ;

m = 9-15 ;
n = 10-18 ;
r = 1 ; et,
W = 1 ou 2.

$A_3$ représente un groupe choisi parmi

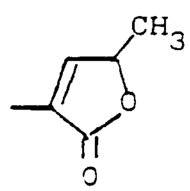

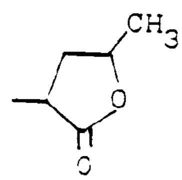

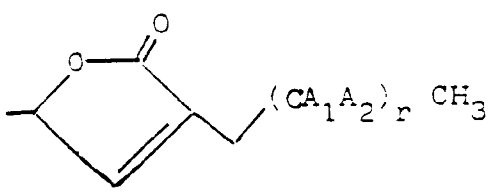 ou

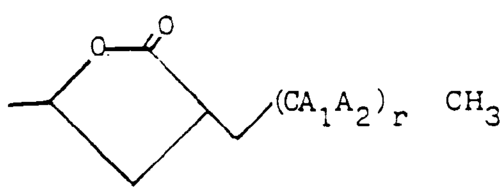 ;

Le composé pesticide préféré selon ledit US-A-4 721 727 est (un mélange de stéréoisomères) extrait de l'écorce et des graines de <u>Asimina</u> <u>triloba</u>, dénommé "asimicine" et a pour formule développée

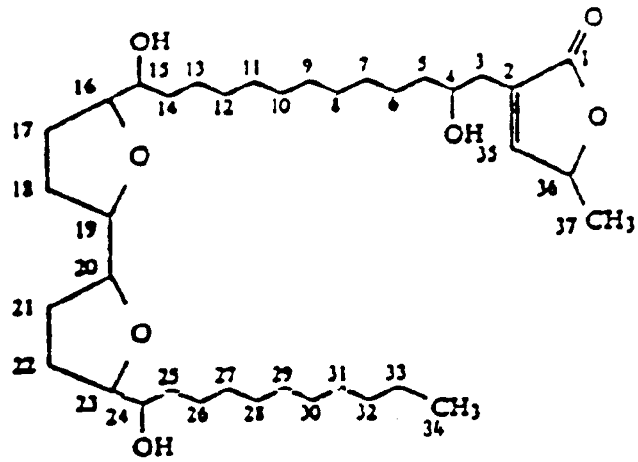

(V)

On connaît de FR-A-2 617 843 l'utilisation de la rolliniastatine 1, qui est un diastéréoisomère de formule V ci-dessus, en tant que substance antitumorale ou antinéoplasique utilisable dans le traitement du cancer.

L'article de G.R. PETTIT ET al., Can. J. Chem., 65 (No 6), pages 1433-1435, (1987) met en évidence, par analyse spectrale RMN, que ladite rolliniastatine 1 est effectivement un diastéréoisomère de l'asimicine de formule V ci-dessus. Voir à cet effet le tableau 2 page 1434 dudit article donnant de façon comparative les spectres RMN de la rolliniastatine 1 et de l'asimicine.

**OBJET DE L'INVENTION**

Selon un premier aspect de l'invention, on préconise une nouvelle utilisation de composés acétogénines, qui sont inclus ou non en tant que produits dans la formule générale (voir formule IV précitée) dudit brevet US-A-4 721 727, en tant que nouvelles substances antiparasitaires.

Plus précisément, on préconise suivant l'invention l'utilisation d'une substance choisie parmi l'ensemble constitué par

(i) les (tétraydrofurannyl)-lactones répondant à la formule

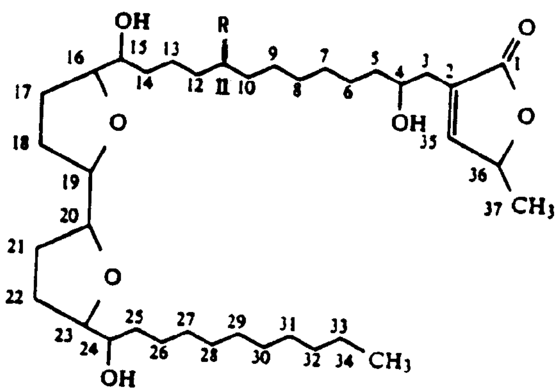

(I)

dans laquelle R représente H ou OH; et

(ii) leurs stéréoisomères optiques et géométriques, pour la préparation d'un médicament antiparasitaire destiné à une utilisation en thérapeutique humaine et vétérinaire vis-à-vis (A) des parasitoses quand R est OH, et (B) des leishmaniose, bilharziose et leptospirose quand R est H.

Lesdites (tétrahydrofurannyl)-lactones sont en général des substances extraites de plantes appartenant à la famille des annonacées.

Selon un second aspect de l'invention, on préconise en tant que produits industriels nouveaux des (tétrahydrofurannyl)-lactones non encore décrites dans l'art antérieur.

Selon un troisième aspect de l'invention, on préconise une composition thérapeutique renfermant comme ingrédient actif au moins un dit composé acétogénine en tant que médicament antiparasitaire.

## DESCRIPTION DETAILLEE DE L'INVENTION

Les acétogénines que l'on utilise suivant l'invention en tant que substances antiparasitaires sont des composés choisis parmi l'ensemble constitué par

– les diastéréoisomères de formule I qui sont tétrahydroxylés (R = OH) en positions 3, 11, 15 et 24 selon la numérotation des atomes de carbone proposée par le premier article précité de JOLAD et al., et qui répondent à la nomenclature systématique de 2-[5-(1-hydroxyundécyl)-2-tétrahydrofurannyl)]-5-[13-(5-méthyl-2-oxo-2,5-dihydro-3-furannyl)-1,5,12-trihydroxytridécyl]-tétrahydrofuranne; et,

– les diastéréoisomères de formule I qui sont trihydroxylés (R = H) en positions 3, 15 et 24 selon ladite numérotation de JOLAD et al., et qui répondent à la nomenclature systématique de 2-[5-(1-hydroxyundécyl)-2-tétrahydrofurannyl]-5-[13-(5-méthyl-2-oxo-2,5-dihydro-3-furannyl)-1,12-dihydroxytridécyl]-tétrahydrofuranne.

Par "stéréoisomères" (ou "diastéréoisomères") on entend ici les isomères optiques qui résultent de la présence de plusieurs atomes de carbone asymétriques, notamment ceux qui comportent un substituant OH, pouvant être de configuration R ou S, d'une part, et les isomères géométriques de constitution qui résultent des configurations respectives (notamment E ou Z) des substituants de chaque cycle, d'autre part.

Les composés préférés, selon l'invention, sur le plan pharmacologique eu égard à leurs propriétés antiparasitaires sont les produits tétrahydroxylés (R = OH) de formule I.

Ces composés tétrahydroxylés sont nouveaux. En revanche, les composés trihydroxylés selon l'invention ont une formule développée connue (voir formule V ci-dessus) et sont très vraisemblablement des diastéréoisomères de la rolliniastatine 1 et de l'asimicine.

Les composés acétogénines selon l'invention peuvent être préparés suivant des modalités opératoires connues en soi par application de mécanismes réactionnels ou extractifs classiques. Le procédé de préparation, que l'on préconise pour l'obtention des composés acétogénines de formule I comprend l'extraction d'une plante, qui est une espèce du genre Rollinia ou une espèce apparentée appartenant à la famille des annonacées, avec au moins un solvant approprié de façon à retenir les fractions donnant une réaction positive avec le réactif de KEDDE, puis isole lesdites fractions.

Le réactif de KEDDE est une solution à 2 % (p/v) d'acide 3,5-dinitrobenzoïque dans l'éthanol. On introduit quelques gouttes de ce réactif dans l'échantillon à tester qui a été préalablement mis en solution dans quelques gouttes de KOH à 6 % p/v dans de l'éthanol. L'apparition d'une coloration rose à violet-intense traduit la présence dans l'échantillon à tester d'une molécule comprenant un reste $\gamma$-lactonique. Voir à cet effet la publication de K.G. KREBBS et al., "Spray Reagents" dans l'ouvrage de E. STAHL "Thin-Layer Chromatography", Springer-Verlag, Berlin, Heideberg, New York, 1969.

Pour l'obtention des composés de formule I où R = H ou OH, on préconise un procédé d'extraction qui est mis en oeuvre à partir d'une plante d'une espèce appartenant au genre Rollinia ou d'une espèce apparentée de la famille des annonacées, préalablement broyée et séchée aux opérations suivantes :

1°) extraction par un solvant choisi parmi l'ensemble comprenant les éthers, les hydrocarbures aliphatiques saturés, les hydrocarbures aliphatiques halogénés, les hydrocarbures alicycliques, les hydrocarbures aromatiques, les hydrocarbures aromatiques halogénés, les alcools en $C_1$-$C_5$, les cétones en $C_3$-$C_7$, le tétrahydrofuranne, le diméthylformamide, les esters et leurs mélanges ;

2°) séparation du milieu d'extraction pour écarter l'insoluble et recueillir le milieu liquide d'extraction ;

3°) concentration sous pression réduite du milieu liquide d'extraction ainsi obtenu ;

4°) lavage du concentrat ainsi obtenu avec un acide minéral dilué 0,5-1,5 M puis de l'eau distillée ;

5°) évaporation du solvant d'extraction contenu dans le concentrat ainsi lavé pour obtenir un résidu substantiellement dépourvu dudit solvant ;

6°) chromatographie dudit résidu ainsi obtenu avec des solvants de polarité croissante, sur colonne de silice, pour ne recueillir que les fractions d'éluat donnant une réaction positive au réactif de KEDDE ;

7°) purification desdites fractions donnant une réaction positive au réactif de KEDDE et séparation des composés de formule I où R = H ou OH.

Dans ce procédé la matière première végétale broyée et séchée est constituée par une partie de la plante (les racines, l'écorce, le bois, les feuilles, les tiges, les graines ou les sommités fleuries) ou la plante entière. De préférence, on utilisera les feuilles ou les graines de Rollinia ou d'une espèce appartenant à la famille des annonacées, préalablement séchées et broyées.

Le genre Rollinia est bien connu. Il comprend notamment des espèces poussant notamment en Amérique du Sud et plus précisément au Pérou et en Equateur, à savoir l'espèce Rollinia ulei [décrite notamment par DIELS, Verh. Bot. Vereins Prov. Brandeburg, 47, 136 (1905)], l'espèce Rollinia microcarpa [décrite notamment par FRIES, Acta Horti Berg., 12 (No 1), 187, (1934)], l'espèce Rollinia ruboides, l'espèce Rollinia papilionella et l'espèce Rollinia sylvatica.

Les espèces apparentées comprennent notamment les Asimina triloba, Uvaria accuminata, Annona cherimolia et Annona densicoma.

La variété préférée selon l'invention appartient à l'espèce Rollinia ulei et est dénommée ci-après la variété Rollinia ulei (MAAS 5972) ou Rollinia sp MAAS (5972). Elle a été décrite par le Professeur P.J.M. MAAS et enregistrée sous le No 5972 du catalogue de l'herbier tenu par l'Institut de Botannique Systématique de l'Université d'Etat d'Utrecht, Pays-Bas.

Pour l'obtention des composés de formule I sus-visés les parties préférées de la plante sont constituées principalement par les feuilles et les graines.

Parmi les solvants d'extraction utilisables pour la mise en oeuvre du stade 1°), on peut citer les éthers (tels que notamment le diméthyléther, le diéthyléther et le diisopropyléther), les hydrocarbures aliphatiques (tels que notamment le pentane, l'hexane, l'heptane et l'éther de pétrole), les hydrocarbures aliphatiques halogénés (tels que notamment le tétrachlorure de carbone, le chloroforme, le dichlorométhane, le 1,1- dichloroéthane, le 1,2-dichloroéthane et le 1,2-dichloroéthylène), les hydrocarbures alicycliques (tels que notamment le cyclopentane et le cyclohexane), les hydrocarbures aromatiques (tels que notamment le benzène, le toluène et les o-, m- et p-toluènes ), les hydrocarbures aromatiques halogénés (tels que notamment le chlorobenzène, les dichlorobenzènes et le bromobenzène), le tétrahydrofuranne, le diméthylformamide, les alcools en $C_1$-$C_5$ (tels que notamment le méthanol, l'éthanol, le n-propanol et l'isopropanol), les cétones en $C_3$-$C_7$, (tels que notamment l'acétone, la méthyléthylcétone, la méthylpropylcétone),les esters (tels que notamment les acétates d'alkyle inférieur en $C_1$-$C_4$, par exemple l'acétate d'éthyle) et leurs mélanges.

Les solvants préférés sont le chloroforme, le dichlorométhane, le méthanol, l'éthanol, les mélanges eau-méthanol et eau-éthanol, l'acétate d'éthyle et (surtout) l'éther de pétrole, ou encore les mélanges desdits solvants.

L'extraction du stade 1°) est mise en oeuvre à une température se situant entre 10°C et la température de reflux du milieu d'extraction notamment à une température se situant dans la gamme de 15°C à 35°C, sous pression atmosphérique (i.e. environ $10^5$ Pa). Le cas échéant, cette extraction peut être réalisée sous pression réduite.

De façon avantageuse on utilisera un volume de solvant d'extraction de 1 litre pour une quantité de 90 à 180 g de matière première végétale broyée et séchée de départ.

La séparation du stade 2°) est une filtration classique qui a pour objet d'écarter l'insoluble et de recueillir l'extrait liquide ou milieu liquide d'extraction. Elle est réalisée notamment par gravité.

La concentration prévue au stade 3°) est réalisée sous pression réduite. De préference cette concentration sera mise en oeuvre de façon à obtenir 0,07 à 0,15 volume de concentrat à partir de 1 volume de solvant d'extraction utilisé au stade 1°), et mieux 0,1 volume de concentrat à partir de 1 volume de solvant d'extraction utilisé au stade 1°). On utilisera notamment un évaporateur rotatif opérant sous pression réduite avec un bain-marie à une température de 40 à 70°C.

Le lavage du stade 4°) est entrepris avec un acide minéral dilué puis avec de l'eau distillée. L'acide minéral dilué peut être HCl, $H_2SO_4$ ou $HNO_3$. De façon avantageuse on utilisera deux fois 1 volume d'acide HCl 0,5-1,5N et mieux 1 volume d'acide HCl 1N pour 4 volumes de concentrat obtenu au stade 3°), puis, au moins 1 volume d'eau distillée pour 4 volumes de concentrat obtenu au stade 3°).

L'évaporation du stade 5°) est avantageusement effectuée sous pression réduite de façon à éliminer pratiquement tout le solvant d'extraction contenu dans le concentrat lavé obtenu après réalisation du stade 4°).

La chromatographie du stade 6°) est effectuée avec des solvants de polarité croissante en vue de retenir les fractions d'éluat donnant une réaction positive au réactif de KEDDE selon les qualités données ci-dessus.

En bref, le stade 6°) comprend une série d'élutions successives. De façon avantageuse ces élutions successives sont réalisées par "chromatographie-éclair" (en anglais : "flash chromatography") c'est-à-dire par chromatographie sur colonne et sous pression, suivant la technique décrite par W.C. STILL et al., J. Org. Chem., 43, pages 2923-2925, (1978). De préférence, la colonne utilisée ici sera garnie de silice de faible granulométrie, par exemple une silice ayant des particules d'un diamètre moyen inférieur ou égal à 80 μm.

De préférence, la purification du stade 7°) comprend une chromatographie-éclair puis une séparation sur plaque de silice.

De façon avantageuse les chromatographies des stades 6°) et 7°) sont mises en oeuvre successivement avec les éluants suivants :

pour le Stade 6°) :

a) un mélange hexane/acétate d'éthyle où les quantités respectives des deux composants sont en proportions variables [convient notamment le mélange hexane/acétate d'éthyle (60/40) v/v],

b) l'acétate d'éthyle pur,

c) un mélange acétate d'éthyle/méthanol où les quantités respectives des deux composants sont en proportions variables [notamment le mélange acétate d'éthyle/méthanol (98/2) v/v],

puis pour le stade 7°) :

$d_1$) un mélange toluène/isopropanol où les quantités respectives des deux composants sont en proportions variables [notamment le mélange toluène/isopropanol (88/12) v/v] pouvant contenir le cas échéant une faible quantité [qui est inférieure ou égale à 5 % en volume par rapport au volume dudit mélange] de diéthylamine,

$d_2$) un mélange chloroforme/acétone/éthanol où les quantités respectives des trois composants sont en proportion variables, ou

$d_3$) un mélange des solvants $d_1$ et $d_2$ en proportions variables.

En variante, on peut utiliser lors de la mise en oeuvre du stade 6°), un ou plusieurs solvants $d_1$, $d_2$ ou $d_3$ ; on utilisera alors au stade 7°) un solvant $d_1$, $d_2$ ou $d_3$ moins polaire.

Par ailleurs, lorsque l'on effectue la chromatographie-éclair des stades 6°) et 7°), l'on recommande avantageusement que la pression de gaz dans la colonne d'élution soit comprise entre 0,2 et 0,25 bars quand l'éluant est un mélange toluène/isopropanol, d'une part, et comprise entre 0,18 et 0,22 bars quand l'éluant ne comporte pas ledit mélange toluène/isopropanol, d'autre part.

Le meilleur mode de mise en oeuvre de ce procédé d'extraction consiste à soumettre les feuilles broyées et séchées de Rollinia sp MAAS (5972) aux opérations suivantes :

1°) extraction avec l'éther de pétrole à une température comprise entre 10°C et la température de reflux du milieu d'extraction (i.e. la température d'ébullition du solvant d'extraction), à raison de 1 litre d'éther de pétrole pour 90 à 180 g de matière végétale broyée et séchée ;

2°) filtration pour écarter l'insoluble et recueillir l'extrait éthéropétrolique ainsi obtenu ;

3°) concentration dudit extrait éthéropétrolique sous pression réduite de façon à obtenir 0,07 à 0,15 volume de concentrat à partir de 1 volume d'éther de pétrole utilisé au stade 1°) ;

4°) lavage du concentrat ainsi obtenu avec de l'acide HCl dilué puis de l'eau, à raison d'au moins deux fois 1 volume d'acide HCl 0,5-1,5N puis d'au moins une fois 1 volume d'eau distillée pour 4 volumes de concentrat obtenu au stade 3°);

5°) évaporation du concentrat ainsi lavé sous pression réduite de façon à éliminer pratiquement tout l'éther de pétrole contenu dans ledit concentrat lavé ;

6°) chromatographie sous pression du résidu d'évaporation ainsi obtenu avec des solvants de polarité croissante sur colonne de silice de granulométrie inférieure ou égale à 80 µm, lesdits solvants comprenant successivement

a) un mélange hexane/acétate d'éthyle où les quantités respectives des deux composants sont en proportions variables [notamment le mélange hexane/acétate d'éthyle (60/40) v/v)],

b) l'acétate d'éthyle pur,

c) un mélange acétate d'éthyle/méthanol où les quantités respectives des deux composants sont en proportions variables [notamment le mélange acétate d'éthyle/méthanol (98/2) v/v], ou des solvants analogues,

de façon à ne retenir que les fractions d'éluat donnant une réaction positive au réactif de KEDDE ;

7°) purification desdites fractions donnant une réaction positive au réactif de KEDDE par chromatographie sous pression sur colonne de silice de faible granulométrie avec comme éluant

$d_1$) un mélange toluène/isopropanol où les quantités respectives des deux composants sont en proportions variables [notamment le mélange toluène/isopropanol (88/12) v/v] pouvant contenir le cas échéant une faible quantité [qui est inférieure ou égale à 5 % en volume par rapport au volume dudit mélange] de diéthylamine,

$d_2$) un mélange chloroforme/acétone/éthanol où les quantités respectives des trois composants sont en proportion variables, ou

$d_3$) un mélange des solvants $d_1$ et $d_2$ en proportions variables,

ou un éluant analogue,

puis séparation par chromatographie sur couche mince de silice pour isoler au moins un des composés de formule I où R = H ou OH.

De façon pratique les stades 1°) et 2°) sont réalisables successivement avec un appareil de type Soxhlet.

Selon l'invention on préconise une composition thérapeutique tant humaine que vétérinaire, qui renferme, en association avec un excipient physiologiquement acceptable, au moins un composé choisi parmi l'ensemble comprenant les composés de formule I et leurs stéréoisomères optiques et géométriques.

Bien entendu dans une telle composition qui convient tant en thérapeutique humaine et vétérinaire, et notamment pour les mammifères, l'ingrédient actif intervient suivant une quantité pharmaceutiquement efficace.

D'autres avantages et caractéristiques de l'invention seront mieux compris à la lecture qui va suivre d'exemples de préparation et de résultats d'essais pharmacologiques. Bien entendu, l'ensemble de ces éléments n'est nullement limitatif mais est donné à titre d'illustration.

EXEMPLE 1

Obtention de 2-[5-(1-hydroxyundécyl)-2-tétrahydrofurannyl]-5-[13-(5-méthyl-2-oxo-2,5-dihydro-3-furannyl)-1,5,12-trihydroxytridécyl]-tétrahydrofuranne

(No de Code : OL 261087)

260 g de feuilles de Rollinia sp MAAS (5972) réduites en poudre et séchées sont extraites par 2 litres d'éther de pétrole dans un appareil de type Soxhlet. La solution éthéropétrolique est concentrée sous pression réduite jusqu'à 200 ml, lavée par 2 fois 50 ml d'acide chlorhydrique 1N, 1 fois par 50 ml d'eau distillée et séchée sur sulfate de sodium anhydre. L'évaporation de l'éther de pétrole laisse un résidu de 11,3 g (rendement : 4,4 %). 8 g de l'extrait éthéropétrolique, en solution dans le mélange hexane/acétate d'éthyle (60/40) v/v sont chromatographiés sous pression, sur 190 g de silice (Kieselgel Merck 9385). L'élution est effectuée par ledit mélange hexane/acétate d'éthyle (60/40) v/v, ensuite par de l'acétate d'éthyle pur puis par le mélange acétate d'éthyle/méthanol (98/2) v/v. On obtient deux fractions A et B donnant une réaction positive au réactif de KEDDE. La fraction A qui est éluée par ledit mélange acétate d'éthyle/méthanol (0,5 g) donne une réaction très positive au réactif de KEDDE. Cette première fraction est rechromatographiée sous pression sur colonne de silice (95 g) en utilisant le solvant toluène/isopropanol (88/12) v/v. On recueille la deuxième fraction d'élution ainsi obtenue, qui est constituée par 200 mg du composé OL 261087 pur [Rf 0,30 par CCM sur plaque de silice; solvant: toluène/isopropanol/diéthylamine, (87,5/10/2,5) v/v]. Le rendement global est de 1,1 g pour 1 kg de matière première végétale broyée et séchée de départ.

**EXEMPLES 2 ET 3**

Obtention de deux isomères du produit de l'exemple I

(Nos de Code : OL 291087 et OL 301087)

La fraction B obtenue lors de la séparation de l'extrait éthéropétrolique suivant les modalités opératoires décrites à l'exemple 1 ci-dessus fournit une réaction positive au réactif de KEDDE. Cette fraction (0,8 g) est rechromatographiée sous pression sur colonne de silice (95 g) en utilisant comme éluant le mélange toluène/isopropanol/acétone (75/6/19) v/v. On obtient plusieurs fractions et recueille les fractions 3 et 5.

La fraction 3 est constituée par 155 mg du composé OL 291087 pur [Rf 0,25 par CCM sur plaque de silice; éluant : toluène/isopropanol/diéthylamine, (87,5/10/2,5) v/v]. Le rendement est de 0,8 g pour 1 kg de matière première végétale broyée et séchée de départ.

La fraction 5 est constituée par 95 mg du composé OL 301087 pur [Rf 0,22-0,23 par CCM sur plaque de silice; éluant : toluène/isopropanol/diéthylamine, (87,5/10/2,5) v/v]. Ce produit se distingue faiblement par CCM du composé OL 291087. Le rendement est de 0,5 g pour 1 kg de matière première végétale broyée et séchée de départ.

**EXEMPLES 4 A 6**

Obtention de trois autres isomères du produit de l'exemple 1

(Nos de Code : OL 161187, OL 171187 et OL 181187)

Après l'extraction par l'éther de pétrole de 260 g de feuilles de Rollinia ulei (MAAS 5972) selon les modalités décrites à l'exemple 1, le marc est séché pendant deux mois sous hotte ventilée. Il est ensuite trituré pendant deux heures avec 30 ml d'ammoniaque diluée au demi. La poudre végétale ainsi alcalinisée est alors extraite par 2,5 litres de dichlorométhane dans un appareil de type Soxhlet. La solution dichlorométhylique est concentrée sous pression réduite dans un évaporateur rotatif jusqu'à occuper un volume de 1 litre. Le concentrat ainsi obtenu est lavé 3 fois par 200 ml d'acide HCl 1N, 2 fois par 200 ml d'eau distillée puis séché sur sulfate de sodium anhydre. Le concentrat lavé ainsi obtenu est débarassé d'une partie de ses pigments par agitation en présence de charbon végétal puis, après filtration sur célite, ledit chlorométhane est évaporé sous pression réduite au moyen d'un évaporateur rotatif. Le résidu (8,5 g) est remis en solution dans le mélange chloroforme/acétone/éthanol (95/4/1) v/v et chromatographié sous pression sur 190 g de silice (Kielselgel Merck 9385). L'élution est effectuée au moyen dudit mélange chloroforme/acétone/éthanol (95/4/1) v/v. Parmi les 16 fractions obtenues, trois présentent des réactions très positives au réactif de KEDDE, à savoir : les fractions 10, 11 et 13.

La fraction 11 (1,5 g) est soumise à une nouvelle chromatographie sous pression sur colonne de silice (112 g) en utilisant le mélange toluène/isopropanol (90/10) v/v. Cette chromatographie permet d'obtenir successivement trois composés purs, à savoir les composés OL 161187 (40 mg), OL 171187 (405 mg) et respectivement OL 181187 (219 mg). Les Rf respectifs sont 0,22 0,20 et 0,15 par CCM sur plaque de silice avec comme éluant le mélange toluène/isopropanol/diéthylamine, (87,5/10/2,5) v/v. Les rendements obtenus pour 1 kg de matière première végétale broyée et séchée de départ sont les suivants : 0,15 g (OL 161187, Ex 4), 1,5 g (OL 171187, Ex 5) et 0,8 g (OL 181187, Ex 6).

**EXEMPLE 7**

Obtention d'un autre isomère du produit de l'exemple 1

(No de Code : OL 301187)

La fraction 13, obtenue lors de la chromatographie de l'extrait dichlorométhylique selon les modalités opératoires décrites aux exemples 4-6, est soumise à une nouvelle chromatographie sous pression sur colonne de silice (112 g) en utilisant comme éluant le mélange toluène/isopropanol (87,5/12,5) v/v. La deuxième fraction que l'on recueille est constituée par 78 mg du composé OL 301187 pur [Rf 0,25 par CCM sur plaque de silice; éluant : toluène/isopropanol/diéthylamine, (87,5/10/2,5) v/v]. Le rendement global est de environ 0,3 g de OL 301187 pour 1 kg de matière première végétale broyée et séchée de départ.

EXEMPLE 8

Obtention de 2-[5-(1-hydroxyundécyl)-2-tétrahydrofurannyl]-5-[13-(5-méthyl-2-oxo-2,5-dihydro-3-furannyl)-1,12-dihydroxytridécyl]-tétrahydrofuranne

(No de Code : OL 051187)

La fraction 10, obtenue lors de la chromatographie de l'extrait dichlorométhylique selon les modalités opératoires décrites aux exemples 4-6, est soumise à une nouvelle chromatographie sous pression sur colonne de silice (112 g) en utilisant comme éluant le mélange toluène/isopropanol (87,5/12,5) v/v. La deuxième fraction que l'on recueille est constituée par 76 mg du composé OL 051187 pur [Rf 0,3 par CCM sur plaque de silice; éluant : toluène/isopropanol/diéthylamine, (87,5/10/2,5) v/v). Le rendement global est de environ 0,3 g de OL 301187 pour 1 kg de matière première végétale broyée et séchée de départ.

ETUDE PHARMACOLOGIQUE

On a résumé ci-après les résultats des essais qui ont été entrepris avec les composés selon l'invention.

La toxicité a été évaluée par la mesure de la cytotoxicité et de la cytostase vis-à-vis de cellules KB selon une technique usuelle. L'activité antiparasitaire a été déterminée in vitro sur Leishmania donovani, promastigote selon un protocole opératoire décrit ci-après.

ETUDE DE CYTOTOXICITE ET CYTOSTASE

Souche testée :

On fait appel à des cellules épithéliales KB provenant d'un carcinome oral épidermoide humain obtenues selon H. EAGLE "Propagation in a fluid medium of a human epidermoid carcinoma strain KB (21811)", Proc. Soc. Exp. Biol. and Med., 89, pages 362-364, (1955) et commercialisées par la Société dite EUROBIO.

Milieu de culture :

Le milieu de culture utilisé avait la composition suivante

- milieu de culture 199 ............... 90 % en volume
- milieu SVF ......................... 10 % en volume
- tampon HEPES (acide N-2-hydroxyéthyl-pipérazine-N'-2-éthanesulfonique) ... 2 ml pour 100 ml de milieu

Mesure de l'effet cytostatique :

L'ensemencement se fait en boîte de 12 cupules contenant chacune 2 ml du milieu. Le produit à tester est mis en solution dans le diméthylsulfoxyde (DMSO) et dilué par du milieu de culture. La solution ainsi obtenue est ajoutée au milieu de culture pour obtenir des concentrations en produit à tester de 5, 10, 20, 30, 40, et 50 µm par ml de milieu. Les cellules sont ajoutées en même temps que la solution du produit à tester.

Le milieu ensemencé et ainsi traité est mis à l'étuve à 37°C pendant 3 jours. Après fixation des cellules par la méthode dite au cristal violet [selon la technique de R.J. GILLIER et al., "Determination of cell number in monolayer cultures", Analytical Biochemistry 159, pages 109-113, (1986)]. La mesure de l'activité cytostatique est effectuée par lecture de la densité optique à 590 nm. La valeur obtenue est rapportée à un témoin dépourvu de produit (densité optique de 100 %).

Mesure de l'effet cytotoxique :

Dans les conditions précédentes, la dose cytotoxique correspond à la dose létale pour 100 % des cellules (disparition complète du tapis cellulaire).

Résultats :

Les résultats obtenus sont consignés dans le tableau I ci-après.

**ETUDE ANTIPARASITAIRE**

Souche testée :

On fait appel à une souche de Lieshmania donovani LRC L52 (de la collection OMS).

Milieu de culture :

Le milieu de culture utilisé avait la composition suivante

- milieu EAGLE modifié DUBBELCO ........ 45 % en volume
- milieu RPMI 1640 contenant 25 % millimolaire de tampon HEPES .............. 45 % en volume
- sérum de veau foetal inactivé à la chaleur ........................ 10 % en volume
- pénicilline à 5 UI/ml ................ 5µg/ml
- kanamycine ......................... 5µg/ml

Mesure de l'effet anti-Leishmania :

L'ensemencement se fait dans des boîtes de 24 cupules contenant chacune 500 µl de milieu. Le milieu est ensemencé par 500 000 promastigotes par cupule puis placé dans une étuve à $CO_2$ à 26°C pendant 3 à 4 heures.

Le produit à tester est ajouté en solution dans le DMSO à des concentrations variables sous un volume de 25 µl par cupule. Les boîtes ensemencées et traitées par le produit sont laissées à l'étuve pendant 3 à 4 jours.

La mesure de l'activité est effectuée par comptage des cellules à l'hémocytomètre, le nombre obtenu étant rapporté à un témoin dépourvu de produit (100 % de cellules, soit 0 % d'inhibition de croissance). Chaque manipulation est effectuée 2 fois.

Résultats :

Les résultats obtenus sont également consignés dans le tableau I ci-après où sont données les doses

d'inhibition à 50 % (DI-50) et les doses d'inhibition maximale mesurées (DImax).

Avec les acétogénines suivant l'invention, on a obtenu de bons résultats dans le traitement de parasitoses, notamment la bilharziose chez l'homme et la leptospirose chez le chien.

**TABLEAU I**

| Produit | No de Code | Cytotoxicité (a) (b) | Cytostase (a) (b) | DI-50 (b) | DImax (I%) (b) |
|---|---|---|---|---|---|
| Ex 1 | OL 261087 | 40 | 20*** | 2,5 | 10 (100%) (c) |
| Ex 2 | OL 291087 | 40 | 20** | 6,2 | 10 (87%) (d) |
| Ex 3 | OL 301087 | 40 | 20** | 10,0 | 20 (79%) |
| Ex 4 | OL 161187 | 40 | 20** | 7,0 | 20 (98%) |
| Ex 5 | OL 171187 | 40 | 20** | 3,0 | 10 (97%) (e) |
| Ex 6 | OL 181187 | 50 | 20** | 7,0 | 10 (71%) (f) |
| Ex 7 | OL 301187 | 40 | 20*** | 5,2 | 10 (96%) (g) |
| Ex 8 | OL 051187 | 40 | 20** | >20 | 20 (11%) |

Notes

- *** statistiquement très significatif
- ** statistiquement significatif
- (a) vis-à-vis de cellules KB
- (b) en µg/ml
- (c) DI = 5 µg/ml pour inhibition de 83 %
- (d) DI = 5 µg/ml pour inhibition de 40 %
- (e) DI = 5 µg/ml pour inhibition de 95 % et DI = 20 µg/ml pour inhibition de 100 %
- (f) DI = 20 µg/ml pour inhibition de 93 %
- (g) DI = 20 µg/ml pour inhibition de 99 %

## Revendications

### Revendications pour les Etats contractants suivants : AT, BE, CH, DE, FR, GB, IT, LI, LU, NL, SE

1. Utilisation d'une substance choisie parmi l'ensemble constitué par

(i) les (tétrahydrofurannyl)-lactones répondant à la formule

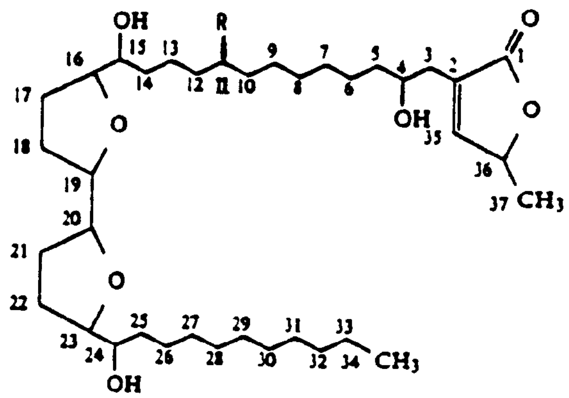

(I)

dans laquelle R représente H ou OH; et

(ii) leurs stéréoisomères optiques et géométriques,

pour la préparation d'un médicament antiparasitaire destiné à une utilisation en thérapeutique humaine et vétérinaire vis-à-vis (A) des parasitoses quand R est OH, et (B) des leishmaniose, bilharziose et leptospirose quand R est H.

2. Utilisation suivant la revendication 1, caractérisée en ce que la substance antiparasitaire est choisie parmi l'ensemble constitué par

(a) le 2-[5-(1-hydroxyundécyl)-2-tétrahydrofurannyl]-5-[13-(5-méthyl-2-oxo-2,5-dihydro-3-furannyl)-1,5,12-trihydroxytridécyl]-tétrahydrofuranne, et

(b) ses stéréoisomères optiques et géométriques.

3. Utilisation suivant la revendication 1, caractérisée en ce que la substance antiparasitaire utile vis-à-vis des leishmaniose, bilharziose et leptospirose est choisie parmi l'ensemble constitué par

(a) le 2-[5-(1-hydroxyundécyl)-2-tétrahydrofurannyl]-5-[13-(5-méthyl-2-oxo-2,5-dihydro-3-furannyl)-1,12-dihydroxytridécyl]-tétrahydrofuranne, et

(b) ses stéréoisomères optiques et géométriques.

4. Composé acétogénine caractérisé en ce qu'il est choisi parmi l'ensemble constitué par

(i) la (tétrahydrofurannyl)-lactone de formule I selon la revendication 1 dans laquelle R représente OH ; et,

(ii) ses stéréoisomères optiques et géométriques.

### Revendications pour les Etats contractants suivants : ES, GR

1. Procédé de préparation d'une acétogénine choisie par l'ensemble constitué par

(i) les (tétrahydrofurannyl)-lactones répondant à la formule

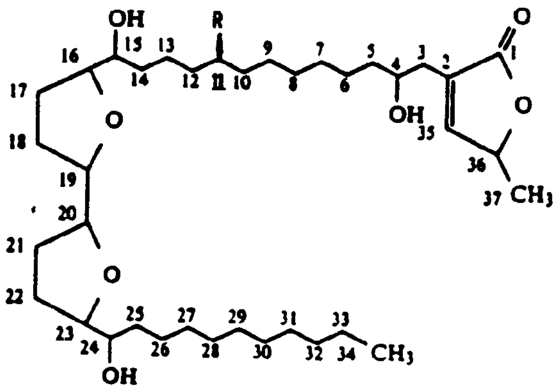

(I)

dans laquelle R représente H ou OH; et

(ii) leurs stéréoisomères optiques et géométri ques;

et utile en thérapeutique humaine et vétérinaire en tant que moyen antiparasitaire, ledit procédé étant caractérisé en ce qu'il consiste à soumettre les feuilles broyées et séchées de la variété Rollinia ulei (MAAS 5972) aux opérations suivantes :

1°) extraction avec l'éther de pétrole à une température comprise entre 10°C et la température de reflux du milieu d'effraction du solvant d'extraction, à raison de 1 litre d'éther de pétrole pour 90 à 180 g de matière végétale broyée et séchée;

2°) filtration pour écarter l'insoluble et recueillir l'extrait éthéropétrolique ainsi obtenu;

3°) concentration dudit extrait éthéropétrolique sous pression réduite de façon à obtenir 0,07 à 0,15 volume de concentrat à partir de 1 volume d'éther de pétrole utilisé au stade 1°);

4°) lavage du concentrat ainsi obtenu avec de l'acide HCl dilué puis de l'eau, à raison d'au moins deux fois 1 volume d'acide HCl 0,5-1,5 N puis d'au moins une fois 1 volume d'eau distillée pour 4 volumes de concentrat obtenu au stade 3°);

5°) évaporation du concentrat ainsi lavé sous pression réduite de façon à éliminer pratiquement tout l'éther de pétrole contenu dans ledit concentrat lavé;

6°) chromatographie sous pression du résidu d'évaporation ainsi obtenu avec des solvants de polarité croissante sur colonne de silice de granulométrie inférieure ou égale à 80 μm, lesdits solvants comprenant successivement

a) un mélange hexane/acétate d'éthyle où les quantités respectives des deux com posants sont en proportions variables,

b) l'acétate d'éthyle pur,

c) un mélange acétate d'éthyle/méthanol où les quantités respectives des deux compo sants sont en proportions variables ou des solvants analogues,

de façon à ne retenir que les fractions d'éluat donnant une réaction positive au réactif de KEDDE;

7°) purification desdites fractions donnant une réaction positive au réactif de KEDDE par chromatographie sous pression sur colonne de silice de faible granulométrie avec comme éluant

$d_1$) un mélange toluène/isopropanol où les quanti tés respectives des deux composants sont en pro portions variables,pouvant contenir, le cas éché ant, une quantité de diéthylamine inférieure ou égale à 5 % en volume par rapport au volume dudit mélange,

$d_2$) un mélange chloroforme/acétone/éthanol où les quantités respectives des trois composants sont en proportion variables, ou

$d_3$) un mélange des solvants $d_1$ et $d_2$ en propor tions variables, ou un éluant analogue,

puis séparation par chromatographie sur couche mince de silice pour isoler au moins un des composés de formule I où R=H ou OH.

2. Procédé suivant la revendication 1, caractérisé en ce que, au stade a), on utilise un mélange hexane/acétate d'éthyle (60/40) v/v.

3. Procédé suivant la revendication 1, caractérisé en ce que, au stade c), on utilise un mélange acétate d'éthyle/méthanol (98/2) v/v.

4. Procédé suivant la revendication 1, caractérisé en ce que, au stade $d_1$), on utilise un mélange toluène/isopropanol (88/12) v/v pouvant contenir en outre une quantité de diéthylamine inférieure ou égale à 5 % en volume par rapport au volume dudit mélange toluène/isopropanol.

5. Procédé suivant la revendication 4, caractérisé en ce que l'on utilise un solvant toluène/isopropanol/ diéthylamine (87,5/10/2,5) v/v.

## Patentansprüche

### Patentansprüche für folgende Vertragsstaaten : AT, BE, CH, DE, FR, GB, IT, LI, LU, NL, SE

1. Verwendung einer Sunstanz ausgewählt aus der Gruppe bestehend aus
(i) den (Tetrahydrofuranyl)-lactonen der Formel

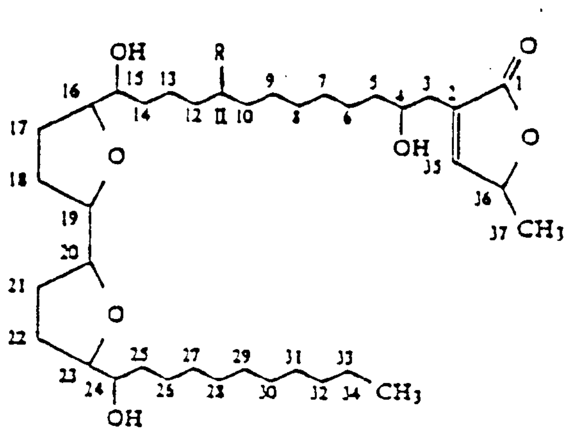

(I)

worin R H oder OH bedeutet; und
(ii) ihren optischen und geometrischen Stereoisomeren,
zur Herstellung eines antiparasitären Arzneimittel zur therapeutischen Verwendung in der Human- und Veterinärmedizin gegenüber (A) Parasitosen, wenn R = OH, und (B) Leishmaniose, Bilharziose und Leptospirose, wenn R = H.

2. Verwendung nach Anspruch 1, dadurch gekennzeichnet, daß die antiparasitäre Substanz ausgewählt ist aus der Gruppe bestehend aus
(a) 2-[5-(1-Hydroxyundecyl)-2-tetrahydrofuranyl]-5-[13-(5-methyl-2-oxo-2,5-dihydro-3-furanyl)-1,5,12-trihydroxytridecyl]-tetrahydrofuran, und
(b) seinen optischen und geometrischen Stereoisomeren.

3. Verwendung nach Anspruch 1, dadurch gekennzeichnet, daß die gegen Leishmaniose, Bilharziose und Leptospirose verwendete antiparasitäre Substanz ausgewählt ist aus der Gruppe bestehend aus
(a) 2-[5-(1-Hydroxyundecyl)-2-tetrahydrofuranyl]-5-[13-(5-methyl-2-oxo-2,5-dihydro-3-furanyl)-1,12-dihydroxytridecyl]-tetrahydrofuran, und
(b) seinen optischen und geometrischen Stereoisomeren.

4. Acetogenin-Verbindung, dadurch gekennzeichnet, daß sie ausgewählt ist aus der Gruppe bestehend aus
i) (Tetrahydrofuranyl)-lacton der Formel I gemäß Anspruch 1, worin R = OH, und
(ii) seinen optischen und geometrischen Stereoisomeren.

### Patentansprüche für folgende Vertragsstaaten : ES, GR

1. Verfahren zur Herstellung eines Acetogenins ausgewählt aus der Gruppe bestehend aus
(i) den (Tetrahydrofuranyl)-lactonen der Formel

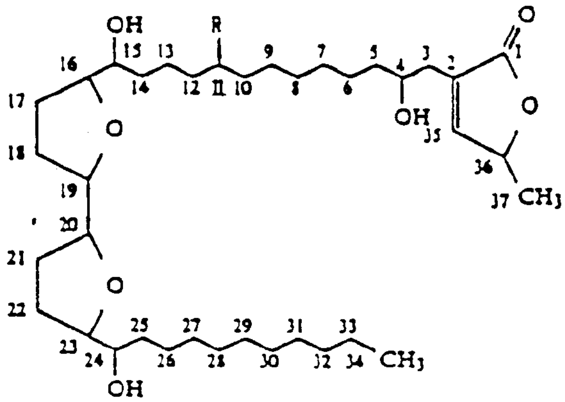

(I)

worin R H oder OH bedeutet; und

(ii) ihren optischen und geometrischen Stereoisomeren;

zur therapeutischen Verwendung in der Human- und Veterinärmedizin als antiparasitäres Mittel, dadurch gekennzeichnet, daß man die zerkleinerten und getrockneten Blätter der Sorte Rollinia ulei (MAAS 5972) den folgenden Verfahrensschritten unterwirft:

1°) Extraktion mit Petrolether bei einer Temperatur zwischen 10°C und der Rückflußtemperatur des Extraktionsmediums des Extraktionsmittels, mit 1 Liter Petrolether für 90 bis 180 g des zerkleinerten und getrockneten pflanzlichen Materials;

2°) Filtration, um Unlösliches abzutrennen und das so erhaltene Petroletherextrakt zu gewinnen;

3°) Aufkonzentrierung des Petroletherextraktes unter vermindertem Druck auf die Weise, daß man 0.07 bis 0.15 Volumenteile Konzentrat aus 1 Volumenteil des in der Stufe 1 verwendeten Petrolethers erhält;

4°) Waschen des so erhaltenen Konzentrates mit verdünnter HCl und dann mit Wasser, wobei man mindestens zweimal 1 Volumenanteil 0.5 bis 1.5 N HCl und dann mindestens einmal 1 Volumenanteil destilliertes Wasser auf 4 Volumenanteile des nach Stufe 3 erhaltenen konzentrates verwendet;

5°) Eindampfen des so gewaschenen konzentrates unter verminderten Druck, um praktisch den gesamten in dem gewaschenen konzentrat enthaltenen Petrolether zu entfernen;

6°) Chromatographie des so erhaltenen Verdampfungsrückstandes unter Druck mit Lösungsmitteln steigender Polarität an einer Säule mit Siliciumoxid einer korngröße von kleiner oder gleich 80 µm, wobei diese Lösungsmittel nacheinanderfolgend umfassen,

a) eine Mischung aus Hexan/Ethylacetat, worin die entsprechenden Mengen der zwei Bestandteile in variablen Anteilen vorhanden sind,

b) reines Methylacetat,

c) eine Mischung aus Ethylacetat/Methanol, worin die entsprechenden Mengen der zwei Komponenten in variablen Anteilen vorhanden sind, oder von analogen Lösungsmitteln,

wobei man nur die Eluatfraktionen zurückbehält, die mit dem KEDDE-Reagens eine positive Reaktion ergeben;

7°) Reinigung dieser Fraktionen, die mit dem KEDDE-Reagens eine positive Reaktion ergeben, durch Chromatographie unter Druck an einer Säule aus Siliciumoxid geringer Korngröße, wobei man als Eluans verwendet

$d_1$ eine Mischung aus Toluol/Isopropanol, worin die entsprechenden Mengen der zwei Bestandteile in variablen Anteilen vorliegen, die gegebenenfalls Diethylamin in einer Menge von kleiner oder gleich 5 Volumen %, bezogen auf die Mischung, enthalten können,

$d_2$ eine Mischung aus Chloroform/Aceton/Ethanol, worin die entsprechenden Mengen der drei Bestandteile in variablen Anteilen vorliegen, oder

$d_3$ eine Mischung der Lösungsmitteln $d_1$ und $d_2$ in variablen Anteilen, oder ein analoges Eluans,

und schließlich eine Trennung durch Chromatographie an einer Siliciumoxid-Dünnschicht durchführt, um mindestens eine der Verbindungen der Formel I, worin R = H oder OH, zu isolieren.

2. Verfahren nach Anspruch 1, dadurch gekennzeichnet, daß man in der Stufe a) eine Mischung aus Hexan/Ethylacetat (60/40) v/v verwendet.

3. Verfahren nach Anspruch 1, dadurch gekennzeichnet, daß man in der Stufe c) eine Mischung aus Ethylacetat/Methanol (98/2) v/v verwendet.

4. Verfahren nach Anspruch 1, dadurch gekennzeichnet, daß man in der stufe $d_1$ eine Mischung aus Toluol/Isopropanol (88/12) v/v verwendet, die außerdem Diethylamin in einer Menge von kleiner oder gleich 5 Vol. %, bezogen auf die Mischung Toluol/Isopropanol, enthalten kann.

5. Verfahren nach Anspruch 4, dadurch gekennzeichnet, daß man als Lösungsmittel Toluol/Isopropanol/Diethylamin (87.5/10/2.5) v/v verwendet.

## Claims

### Claims for the following Contracting States : AT, BE, CH, DE, FR, GB, IT, LI, LU, NL, SE

1. The use of a substance selected from the group consisting of
(i) the (tetrahydrofuryl)-lactones of the formula

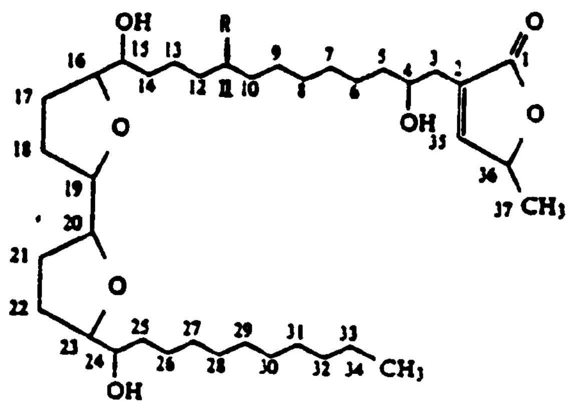

(I)

wherein R is H or OH ; and,
(ii) their optical and geometric stereoisomers
for the preparation of an antiparasitic medicament destined for a human and veterinary therapeutical use vis-a-vis (A) parasitoses when R is OH, and (B) leishmaniosis, bilharziosis and leptospirosis when R is H.

2. The use according to claim 1 wherein the antiparasitic substance is selected from the group consisting of
(a) 2-[5-(1-hydroxyundecyl)-2-tetrahydrofuranyl]-5-[13-(5-methyl-2-oxo-2,5-dihydro-3-furanyl)-1,5,12-trihydroxytridecyl]-tetrahydrofuran, and
(b) optical and geometric stereoisomers thereof.

3. The use according to claim 1 wherein the antiparasitic substance useful vis-a-vis leishmaniosis, bilharziosis and leptospirosis is selected from the group consisting of
(a) 2-[5-(1-hydroxyundecyl)-2-tetrahydrofuranyl]-5-[13-(5-methyl-2-oxo-2,5-dihydro-3-furanyl)-1,12-dihydroxytridecyl]-tetrahydrofuran, and
(b) optical and geometric stereoisomers thereof.

4. An acetogenin compound characterized in that it is selected from the group consisting of
(i) the (tetrahydrofuryl)-lactone of the formula I according to claim 1, wherein R is OH ; and,
(ii) optical and geometric stereoisomers thereof.

### Claims for the following Contracting States : ES, GR

1. A method for preparing an acetogenin compound selected from the group consisting of
(i) the (tetrahydrofuryl)-lactones of the formula

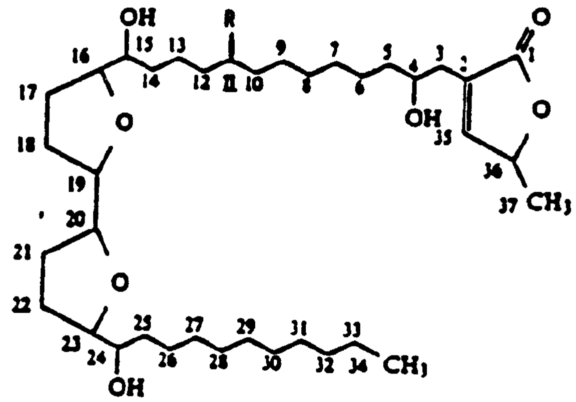

(I)

wherein R is H or OH ; and,

(ii) their optical and geometric stereoisomers ;

and useful in human and veterinary therapy, characterized in that it consists in subjecting the ground and dried leaves of the variety Rollinia ulei (MAAS 5972) to the following operations :

1°) extraction with petroleum ether at a temperature between 10°C and the reflux temperature of the extraction medium, at the rate of 1 liter of petroleum ether per 90 to 180 g of ground and dried plant material ;

2°) filtration to remove the insoluble material and retain the petroleum ether extract thus obtained ;

3°) concentration of the said petroleum ether extract under reduced pressure so as to obtain 0.07 to 0.15 volume of concentrate from 1 volume of petroleum ether used in step 1°) ;

4°) washing of the resultant concentrate with dilute HCl acid, then with water, at the rate of at least twice 1 volume of 0.5-1.5N HCl acid then at least once 1 volume of distilled water per 4 volumes of concentrate thus obtained in step 3°) ;

5°) evaporation of the concentrate thus washed under reduced pressure so as to remove practically all the petroleum ether contained in the said washed concentrate ;

6°) chromatography under pressure of the eva poration residue thus obtained over a silica column wherein the silica has a granalometry lower than or equal to 80 micrometers, with solvents of increasing polarity, said solvents comprising successively :

a) a hexane/ethyl acetate mixture in which the respective amounts of the two components are in variable proportions,

b) pure ethyl acetate,

c) an ethyl acetate/methanol mixture in which the respective amounts of the two components are in variable proportions or analogous solvents,

so as to retain only the eluate fractions producing a positive reaction to the KEDDE reagent ;

7°) purification of the said fractions producing a positive reaction to the KEDDE reagent by chromatography under pressure over a silica column containing silica of small-particle size by using as eluent ;

$d_1$) a toluene/isopropanol mixture in which the respective amounts of the two components are in variable proportions that may possibly contain a diethylamine amount lower than or equal to 5% by volume with respect to the volume of said mixture,

$d_2$) a chloroform/acetone/ethanol mixture in which the respective amounts of the three components are in variable proportions, or

$d_3$) a mixture of the solvents $d_1$ and $d_2$ in variable proportions, or an analogous eluent,

then separation by thin layer chromatography on silica to isolate at least one of the compounds of formula 1 wherein R is H or OH.

2. A method according to claim 1 wherein in step a) a hexane/ethyl acetate (60/40) v/v mixture is used.

3. A method according to claim 1 wherein in step c) an ethyl acetate/methanol (98/2) v/v mixture is used.

4. A method according to claim 1 wherein in step $d_1$) a toluene/isopropanol (88/12) v/v mixture, which can contain a diethylamine amount lower than or equal to 5 % by volume of said toluene/isopropanol mixture, is used.

5. A method according to claim 4 wherein a toluene/isopropanol/diethylamine (87.5/10/2.5) v/v mixture is used as solvent.